# FASCICULE DE BREVET EUROPEEN

(11) **EP 1 040 816 B1**
(45) Date de publication et mention de la délivrance du brevet: **27.03.2002**
(21) Numéro de dépôt: 00400753.0
(22) Date de dépôt: 17.03.2000
(51) Int. Cl.: A61K 7/06, A61K 7/48

(54) **Utilisation cosmétique de particules d'organopolysiloxane reticulé à groupement oxalkyléné dans une composition de maquillage**
Kosmetische Verwendung von teilchenförmigem vernetzten Organopolysiloxan, welches Oxalkylengruppen enthält, in Make-up Zusammensetzungen
Cosmetic use of a particulate crosslinked organopolysiloxane with oxalkylene groups in make-up compositions

(30) Priorité: 30.03.1999 FR 9903967
(43) Date de publication de la demande: 04.10.2000
(73) Titulaire: L'OREAL, 75008 Paris (FR)
(72) Inventeur: Bara, Isabelle, 75013 Paris (FR)
(74) Mandataire: Lhoste, Catherine

(56) Documents cités:
- EP-A- 0 790 055
- US-A- 5 387 417
- US-A- 5 412 004

## Description

La présente invention se rapporte à une composition de soin et/ou de maquillage de la peau et/ou des lèvres des êtres humains présentant à la fois des propriétés matifiantes durables dans le temps et de fraîcheur. Cette composition est en particulier un rouge à lèvres, un eye liner, un fard à joues ou à paupières, un fond de teint, ou encore un produit solaire, un déodorant, un shampooing traitant. Elle se présente notamment sous forme de gel anhydre, de lotion ou de crème plus ou moins épaisse, de produit coulé en stick ou en coupelle.

L'invention se rapporte aussi à l'utilisation de particules d'organopolysiloxane particulier en vue de matifier la peau ou les lèvres et/ou camoufler leurs imperfections.

Les compositions de rouge à lèvres et de fond de teint connues comprennent généralement des corps gras tels que des huiles, des composés pâteux et des cires, ainsi qu'une phase particulaire généralement composée de charges et de pigments. Les charges servent généralement à modifier la texture de la composition ainsi qu'à matifier le film de composition déposé sur la peau et/ou les lèvres alors que les pigments servent généralement à apporter de la couleur à la composition.

L'effet de matité est particulièrement recherché pour les utilisateurs à peaux mixtes ou grasses, ainsi que sous les climats chauds et humides. Les charges matifiantes utilisées sont le plus souvent des charges absorbantes, en particulier du sébum et d'huile excédentaire apportée par la composition, comme le talc, la silice, le kaolin, la poudre de nylon ou encore des charges présentant des propriétés optiques de diffusion de la lumière, propriétés connues sous le nom d'effet "soft focus". Ces charges ont tendance à dessécher la peau, marquer le relief cutané, en particulier les rides, pores, accentuant ainsi les imperfections locales. De plus, les charges absorbantes confèrent à la peau un aspect poudreux peu naturel et ont un effet matifiant peu durable dans le temps.

Plus récemment, on a utiliser des polymères matifiants (voir en particulier le document EP-A-790055) tels que des polymères siliconés réticulés connus sous les références commerciales KSG (KSG 6, 16, 17, 18) de la société Shin Etsu, Tréfils de la société Dow Coming ou Gransils pour la société Grand Industrie.

L'inconvénient de ces produits commerciaux est d'apporter un effet huileux, gras, sans effet frais, ce qui ne permet pas ou difficilement leur utilisation en milieu chaud et humide et/ou par les utilisateurs à peaux grasses. De plus ces produits commerciaux, même ceux exempts d'huile de silicone (Tréfils 505 C de Dow Coming par exemple), sont difficilement dispersibles dans un milieu aqueux. Ces produits sont présentés comme des polymères siliconés élastomèriques « insolubles dans l'eau » (voir notamment le document de Kao EP-A-0855178.

Ces polymères difficilement incorporables en phase aqueuse sont totalement hydrofuges. Du fait de leur forte incompatibilité avec l'eau et en particulier avec la sueur, cette dernière n'est pas absorbée par ces polymères et a même tendance à « perler » à la surface de la peau, lorsque celle-ci transpire. Le pouvoir matifiant de ces polymères a donc tendance à s'estomper au cours du temps. De plus, les compositions les contenant comme les émulsions eau-dans-huile ou huile-dans-eau se déstabilisent au cours du temps.

Récemment, on a conçu des émulsions contenant ce type de polymère (cf. brevets US-A-5 421 004 de Kose et US-A-5 599 533 d'Estée Lauder) en vue d'améliorer leurs propriétés cosmétiques. Ces émulsions, bien qu'apportant moins de gras et plus de fraîcheur que les produits anhydres, perdent leur propriété matifiante initialement apportée par les polymère siliconés réticulés.

Bien qu'il existe des composés de types organosiloxanes réticulés se dispersant en milieu aqueux, comme par exemple, les composés de type KSG 20 ou KSG 21 vendus par la société Shin Etsu, et dont la structure chimique particulière (cf. brevet US-A-5236986 de Shin Etsu) est responsable de cette dispersion en milieu aqueux (présence de groupements polaires leur conférant des propriétés tensioactives), ces composés, contrairement à ceux de la composition de l'invention, n'apportent pas d'effet mat particulier et ne sont pas capables d'émulsionner de forte quantité d'eau (à savoir jusqu'à 70 % en poids d'eau).

Il subsiste donc le besoin d'une composition matifiante stable dans le temps, dont les propriétés persistent sur la peau au cours du temps, et qui apportent en même temps de la fraîcheur.

Par ailleurs, les compositions de fond de teint et/ou de rouge à lèvres connues, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué sur la peau ou les lèvres nécessitant de renouveler régulièrement l'application de la composition de fond de teint ou de rouge à lèvres. Par ailleurs, l'apparition de ces traces inacceptables notamment sur les cols de chemisier peut écarter certaines femmes à utiliser ce type de maquillage.

Depuis plusieurs années, les cosméticiens se sont intéressés aux compositions de rouge à lèvres et de fond de teint « sans transfert ». Ainsi, la société Shiseido a envisagé dans sa demande de brevet JP-A-61-65809 des compositions de rouge à lèvres « sans transfert » contenant de 1 à 70% en poids d'une résine siloxysilicate (à réseau tridimensionnel) comportant des chaînes pendantes alkylées de 1 à 6 atomes de carbone ou phénylées, de 10 à 98% en poids d'une huile de silicone volatile à chaîne silicone cyclique et des charges pulvérulentes. De même la société Noevier à décrit dans le document JP-A-62-61911 des compositions de rouge à lèvres, d'eye liner, de fonds de teint « sans transfert » comportant une ou plusieurs silicones volatiles associées à une ou plusieurs cires hydrocarbonées.

Ces compositions, bien que tout à fait satisfaisantes pour la propriété de « sans transfert » présentent l'inconvénient de laisser sur les lèvres, après évaporation des huiles de silicone, un film qui devient inconfortable au cours du temps (sensation de dessèchement et de tiraillement), écartant un certain nombre de femmes de ce type de produit de maquillage. Pour améliorer le confort de ce type de composition, on pourrait ajouter des huiles non volatiles, mais dans ce cas on perdrait en efficacité « sans transfert ».

Plus récemment, la société Revlon a envisagé dans sa demande de brevet EP-A-602905 des rouges à lèvres « sans transfert » contenant une silicone volatile cyclique ou linéaire à chaînes méthyle pendantes et une résine de silicone comportant une chaîne estérifiée pendante ayant de 12 à 18 atomes de carbone. Le film de rouge à lèvres, restant sur les lèvres après évaporation de la silicone volatile, a encore l'inconvénient de manquer de confort à l'application et notamment d'être trop sec. Elle a, en outre, envisagé dans sa demande de brevet EP-A-709 083 des fonds de teint « sans transfert » contenant une silicone volatile associée à une résine siloxysilicate. Ces fonds de teint présentent encore l'inconvénient d'être peu confortables et secs au cours du temps.

De plus, les compositions de fond de teint ou de rouge à lèvres, lorsqu'elle sont appliquées sur la peau ou les lèvres, et notamment les pigments de ces compositions ont tendance à migrer au cours du temps, c'est-à-dire à se propager à l'intérieur des rides et des ridules de la peau qui entourent les lèvres et les yeux, et à déborder des lèvres, hors du tracé initial entraînant un effet inesthétique. L'apparition de ces traces ont tendance à écarter certaines femmes de l'utilisation de ce type de maquillage.

La présente invention a justement pour objet une composition de soin ou de maquillage de la peau et des lèvres des êtres humains permettant de remédier aux différents inconvénients mentionnés ci-dessus et permettant en particulier l'obtention d'un film ne transférant pas, présentant des propriétés cosmétiques améliorées par rapport à celles des produits « sans transfert » de l'art antérieur, notamment des propriétés de glissant, de non tiraillement et de non dessèchement des lèvres, ainsi que des propriétés matifiantes et de fraîcheur supérieures à celles des produits matifiants de l'art antérieur, propriétés durables dans le temps. Elles présentent, en outre, des propriétés de bonne tenue dans le temps en particulier de la couleur (non virement au cours du temps) et de non migration notamment dans les replis de la peau.

L'invention s'applique non seulement aux produits de maquillage des lèvres et de la peau des êtres humains mais aussi aux produits de soin et/ou de traitement des lèvres et de la peau humaine. La composition de l'invention peut aussi s'appliquer sur le cuir chevelu.

Ainsi, l'invention a pour objet l'utilisation de particules d'organopolysiloxane solide élastomérique réticulé comportant au moins un groupement oxyalkyléné et plus spécialement oxyéthyléné dans une composition, telle que définie dans la revendication 1.

Par imperfections, on entend tout défaut de la peau comme le microrelief (pores, rides, ridules), les tâches et les points noirs, les microvaisseaux sanguins.

Par « élastomérique » on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes de la composition de l'invention contiennent un ou plusieurs groupements oxyalkylénés et en particulier oxyéthylénés (OE), par exemple de 1 à 40 motifs oxyalkylénés et de préférence de 1 à 20 et mieux de 12 à 20, pouvant former des chaînes polyoxyalkylènes et notamment polyoxyéthylènes. Ces groupements peuvent être pendant, en bout de chaîne ou destinés à lier deux parties de la structure siliconée. Le nombre de silicium portant ces groupements sont avantageusement au nombre de 1 à 10 et mieux de 1 à 6.

Bien que l'invention concerne plus spécialement les organopolysiloxanes à groupement(s) oxyéthyléné(s) (à savoir ne comportant que des groupements oxyéthylénés comme groupements oxyalkylénés), elle peut aussi concerner les organopolysiloxanes à groupement(s) oxypropyléné(s), c'est-à-dire ne comportant que des groupements oxypropylénés comme groupements oxyalkylénés. Les organopolysiloxanes peuvent aussi comporter à la fois un ou plusieurs groupement(s) oxyéthyléné(s) 1 à 20 (OE) par exemple et un ou plusieurs groupement(s) oxypropyléné(s) (OP) 0 à 20 par exemple ; ces organopolysiloxanes sont aussi appelés des organopolysiloxanes à groupement(s) alkyléthoxy-propyléné(s). De préférence le nombre de groupements oxyéthylénés est supérieur au nombre de groupements oxypropylénés.

Par ailleurs, la structure siliconée formant le squelette polymérique de l'organopolysiloxane est avantageusement une structure polyméthylsiloxane (PDMS) dont éventuellement une partie des groupes méthyle est substituée par des groupements alkyles en C₂ à C₃₀ et de préférence en C₈ à C₂₄, et mieux de C₁₀ à C₂₀ ou phényle, soit en bout de chaîne soit pendants.

Par ailleurs, l'organopolysiloxane à groupement(s) oxyalkyléné(s) peut comporter un ou plusieurs squelette(s) siliconé(s) relié(s) entre eux par un ou plusieurs groupements oxyalkylénés et de préférence oxyéthylénés tels que définis précédemment ou par un ou plusieurs groupements alkylénés, le nombre de groupement alkyléné allant de 1 à 20 et de préférence de 1 à 10. De préférence, il comporte au moins deux squelettes polymériques liés entre eux.

Avantageusement, le ou les squelettes siliconés des organopolysiloxanes élastomériques de la composition selon l'invention contiennent de 26 à 80 atomes de silicium.

Selon l'invention, les particules d'organopolysiloxanes servent d'émulsifiant de phase aqueuse dans une phase grasse liquide.

Les organopolysiloxanes élastomériques de la composition de l'invention présentent un remarquable pouvoir épaississant de phase grasse liquide et d'émulsification d'une phase grasse liquide dans une phase aqueuse et vise-versa ; ils gonflent dans la phase grasse liquide. Ils ne sont pas desséchants pour la peau et apportent de bonnes propriétés cosmétiques, notamment de douceur, de fraîcheur, de matité, de non gras et de sans transfert. Ces nouveaux élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux huileux et en particulier ceux de la composition et ceux sécrétés par la peau.

La propriété de matifier la peau des compositions selon l'invention est remarquable, intrinsèque et spécifique de ces organopolysiloxanes à groupement(s) oxyalkyléné(s) et notamment oxyéthyléné(s). Avantageusement, la composition est exempte de charge matifiante. La composition de l'invention peut se présenter sous forme de pâte, de solide, de crème plus ou moins fluide. Elle peut être une émulsion huile-dans-eau ou eau-dans-huile plus ou moins fluide, un gel anhydre, solide ou souple, une émulsion multiple et notamment une émulsion eau-dans-huile-dans-eau ou huile-dans-eau-dans-huile. Cette composition peut avoir l'aspect d'une lotion, d'un gel, d'une crème, d'un produit coulé et même se présenter sous forme d'aérosol.

Avantageusement, la composition de l'invention est une émulsion simple ou multiple et est exempte de tensio-actif. De préférence, cette composition contient une grande quantité d'eau, supérieure à 70 % du poids total de la composition.

La composition selon l'invention est stable, c'est-à-dire qu'elle ne démixe pas à température ambiante pendant au moins 2 mois.

Les organopolysiloxanes élastomériques conformes à la composition selon l'invention sont partiellement ou totalement réticulés et de structure tridimensionnelle. Indus dans une phase huileuse, ils se transforment, selon le taux de phase huileuse utilisé, d'un produit d'aspect spongieux lorsqu'ils sont utilisés en présence de faibles teneurs en phase huileuse en un gel homogène, en présence de quantités de phase huileuse plus élevées. La gélification de la phase huileuse par ces élastomères peut être totale ou partielle.

Les élastomères de la composition selon l'invention se présentent sous forme de poudre ou de gel émulsionné contenant un organopolysiloxane élastomère de structure tridimensionnelle, dispersé dans une phase grasse liquide.

Par phase grasse liquide, appelée encore phase huileuse, on entend tout corps non aqueux ou mélange de corps non aqueux, liquides à température ambiante (25°C) et pression atmosphérique (760 mm de Hg).

Les organopolysiloxanes élastomériques de la composition selon l'invention peuvent être choisis parmi les polymères réticulés obtenus par réaction d'addition et de réticulation en milieu non aqueux, en présence d'un catalyseur notamment du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins groupement oxyalkylène notamment oxyéthyléné.

En particulier, l'organopolysiloxane (I) est choisi parmi les polydiméthylsiloxanes (PDMS) et est plus spécifiquement un α-ω-diméthylvinyl polydiméthylsiloxane. L'organopolysiloxane (ii) est choisi notamment parmi les polydiméthylsiloxanes comportant un ou plusieurs atome(s) d'hydrogène, lié chacun à un atome de silicium et un ou plusieurs groupements oxyéthylénés et éventuellement un ou plusieurs groupements oxypropylénés liés, à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Les organopolysiloxanes élastomériques de la composition selon l'invention se présente avantageusement sous forme de gel anhydre. Ce gel peut être notamment obtenu comme suit :
- (a) mélange du premier organopolysiloxane (i) et du second organopolysiloxane (ii) ;
- (b) ajout de la phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à température ambiante (25°C) choisies parmi les huiles hydrocarbonées et/ou les huiles siliconées. Avantageusement, la phase huileuse est une phase liquide siliconée, contenant une ou plusieurs huiles choisies parmi les PDMS à chaîne linéaire ou cyclique liquides à température ambiante comportant éventuellement une chaîne alkyle ou aryle pendante ou en bout de chaîne, la chaîne alkyle ayant de 1 à 6 atomes de carbone.

Par huiles hydrocarbonées, on entend des huiles contenant majoritairement des atomes de carbone et des atomes d'hydrogène et en particulier des chaînes alkyle ou alcényle comme les alcanes, les alcènes. Ces huiles peuvent, en outre, comporter un ou plusieurs groupes ester, éther, hydroxyle ou carboxylique.

Les organopolysiloxanes de la composition selon l'invention sont en particulier obtenus selon le mode opératoire des exemples 3, 4 et 8 du document US-A-5412004 et des exemples du document US-A-5811487. De préférence, on utilise l'organopolysiloxane de l'exemple 3 du brevet US-A-5412004.

Le produit de l'exemple 3 du document US-A-5412004 se présente sous la forme d'un gel pâteux contenant environ 33 % en poids d'organopolysiloxane réticulé à groupement(s) oxyéthyléné(s) et environ 67 % de PDMS 6cSt. L'organopolysiloxane contient environ 18 % du poids total du polymère d'oxyde d'éthylène.

Le gel élastomérique de la composition selon l'invention a un comportement rhéofluidifiant plastique de viscosité dynamique sous faible cisaillement voisin de 10⁻³s⁻¹ ou 10⁻⁴s⁻¹, allant de 2.10⁶P à 4-10⁶P et une viscosité dynamique de 15 à 50 P (1,5 à 5 Pa.s) pour une vitesse de cisaillement de 200s⁻¹ à t 10 min, mesurée avec un rhéomètre à contrainte imposée, RS 75 (Haake) à 25°C en géométrie cône/plan ; caractéristique du cône : 20mm de diamètre, 1° d'angle et 40 µm de gap. L'organopolysiloxane de la composition selon l'invention a, en outre, un comportement visco-élastique à 1Hz avec un caractère élastique dominant aux faibles valeurs de la contrainte de cisaillement défini comme suit : 800 Pa < G * ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité. Il présente un point éclair d'environ 160 à 170°C à pression atmosphérique. Pour le gel élastomérique de l'exemple 3, du document US-A-5412004, la viscosité dynamique est de 45 P (4,5 Pa.s) à la vitesse de cisaillement de 200s⁻¹.

Ce gel élastomérique est stable à température ambiante (25°C) au moins 4 mois (sans exsudation d'huile).

De façon préférentielle, ce gel d'organopolysiloxane élastomérique est présent dans la composition à un taux de 0,5 à 99% et mieux de 3 à 75%, ce qui correspond à un taux de polymère en matière active de 0,1 à 33% en poids et mieux de 1 à 25%.

En particulier, les particules d'organopolysiloxane élastomérique (en matière active) ont une taille allant de 0,1 à 500 µm et mieux de 3 à 200 µm et encore mieux de 3 à 50 µm. Ces particules peuvent être sphériques, plates ou amorphes avec, de préférence, une forme sphérique.

L'organopolysiloxane élastomérique de la composition selon l'invention est en particulier un tensioactif de HLB (Balance Hydrophile Lipophile) d'environ 2,5. Il est donc parfaitement adapté à la fabrication d'émulsion eau-dans-huile stable et d'émulsion huile-dans-eau-dans-huile ou eau-dans-huile-dans-eau, stable.

A ce gel d'organopolysiloxane élastomérique peuvent être associés des corps gras liquides à température ambiante, des cires ou des gommes solides à température ambiante, des corps gras pâteux, d'origine animale, végétale, minérale ou synthétique et leurs mélanges.

La phase grasse additionnelle peut être quelconque et contenir des produits liquides à température ambiante comme les huiles siliconées, fluorées, fluorosiliconées, hydrocarbonées éventuellement partiellement siliconées. Ces huiles pewent être volatiles à température ambiante et pression atmosphérique. Par huile volatile, on entend en particulier une huile susceptible de s'évaporer, en moins d'une heure, au contact de la peau ou des lèvres ayant notamment une pression vapeur non nulle, en particulier allant de 0,13 Pa (10⁻³ mm de Hg) à 40 000 Pa (300 mm de Hg) (à température ambiante et pression atmosphérique) et de préférence supérieure à 40 Pa (0,3 mm de Hg).

Ces huiles peuvent représenter de 1 à 80 % du poids total de la composition, de préférence de 1 à 50 % et mieux de 1 à 30 %

Comme huiles utilisables dans la composition de l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- des alcools gras comme l'octyl dodécanol ou l'alcool oléique ;
- les huiles fluorées partiellement hydrocarbonées et/ou siliconées comme les perfluoropolyesters ;
- les huiles siliconées comme les polyméthylsiloxanes à chaîne siliconée linéaire ou cyclique, liquides ou pâteux à température ambiante, les phényl diméthicones, les phényl triméthicones et les polyméthylphénylsiloxanes, les alkylpolydiméthylsiloxanes avec une chaîne alkyle en C₂ à C₂₀.
- leurs mélanges.

Le gel d'organopolysiloxane à groupement(s) oxyéthyléné(s) permet de structurer ces huiles sous forme d'une texture originale type « flan », exempte de gélifiant huileux qui entraverait le toucher doux/soyeux et agréable de la composition.

Avantageusement, la composition selon l'invention peut contenir au moins une cire choisie parmi des cires hydrocarbonées, fluorées, siliconées et leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion commençante supérieure à 25 °C et mieux supérieure à 45 °C, à pression atmosphérique.

Selon l'invention, une cire est un corps gras lipophile, solide à température ambiante, à changements d'état solide/liquide réversible, ayant une température de fusion commençante pourvant aller jusqu'à 200°C et présentant à l'état solide une organisation cristalline anisotrope. En portant la cire à sa température de fusion, il est possible de la rendre miscible à la phase grasse liquide et de former un mélange homogène microscopiquement, puis en ramenant la température du mélange à la température ambiante, on obtient une cristallisation de la cire dans la phase grasse liquide du mélange.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autre cires utilisables dans l'invention, on peut citer les cires d'origine animale comme la lanoline, la cire d'abeilles ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthylène.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de ciré et de préférence de 5 à 30%.

La composition de l'invention contient avantageusement une phase aqueuse liquide comportant en particulier de l'eau et des solvants miscibles à l'eau en toute proportion comme les polyols (glycérol, diglycérine, éthylène glycol), les monoalcools inférieurs en C₂ à C₅, l'acétone, et la dicétone. La phase aqueuse peut représenter de 0 à 75 % du poids total de la composition et mieux de 5 à 50 %.

La composition de l'invention peut comprendre, en outre, tout additif usuellement utilisé dans le domaine concerné, tel que des colorants hydrosolubles ou liposolubles, des antioxydants, des huiles essentielles, des conservateurs, des actifs cosmétiques ou dermatologiques, des polymères liposolubles notamment hydrocarbonés tels que les polyalkylènes, des gélifiants de phase aqueuse, des gélifiants de phase grasse, les parfums, les électrolytes comme les sels inorganiques divalents ou monovalents (NaCI, MgCl₂, MgSO₄).

Ces additifs peuvent être présents dans la composition selon les quantités habituellement utilisées et par exemple à raison de 0 à 20% du poids total de la composition et mieux de 0,1 à 10%. Pour les électrolytes, on utilise en particulier au moins 30 à 60 milliosmoles.

Avantageusement, la composition de l'invention contient comme additifs un ou plusieurs gélifiants de phase aqueuse, à savoir des composés capables de donner l'aspect d'un gel à la composition et de l'épaissir. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose, la carboxyéthylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de rhamsan, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol » ou «Carbomer» de la Société Goodrich (Carbomer 980 par exemple neutralisé par de la triéthanolamine -TEA en abréviation-) ; les polymères poly(métha)crylates de glycéryle ; la polyvinyipyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs.

Selon l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort, la matité, la fraîcheur, à la non migration et au « non transfert » de la composition.

La composition selon l'invention peut se présenter sous la forme d'un produit coloré et notamment de maquillage de la peau, en particulier un fond de teint, un fard à joues ou à paupières, un eye-liner, un stick anti-cemes, un produit de maquillage du corps (tatouage), un maquillage des phanères comme un mascara, un vernis à ongles ou de maquillage des lèvres comme un rouge à lèvres. Elles peuvent également se présenter sous forme non colorée, contenant éventuellement des actifs cosmétiques ou dermatologiques. Elle peut alors être utilisée comme base de soin pour les lèvres (baumes à lèvres, protégeant les lèvres du froid et/ou du soleil et/ou du vent) ou base fixante à appliquer sur un rouge à lèvres classique, (la base fixante formant alors un film protecteur sur le film de rouge, qui en limite le transfert).

La composition de l'invention peut également se présenter sous la forme d'une composition dermatologique ou cosmétique de traitement ou de soin de la peau (y compris le cuir chevelu), de fibres kératiniques (cheveux, cils, sourcils), des ongles ou des lèvres, ou sous forme d'une composition de protection solaire ou de bronzage artificiel, ou encore sous forme d'un produit nettoyant ou démaquillant de la peau ou des fibres kératiniques, un produit déodorant.

Bien entendu la composition de l'invention doit être cosmétiquement ou dermatologiquement acceptable, à savoir non toxique et susceptible d'être appliquée sur la peau (y compris l'intérieur des paupières) ou les lèvres d'êtres humains. Par cosmétiquement acceptable, on entend au sens de l'invention, une composition d'aspect, d'odeur et de toucher agréables.

De façon préférentielle, la composition de l'invention peut comprendre une phase particulaire, généralement présente à raison de 0 à 60 % du poids total de la composition, de préférence de 5 à 35 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 60 % du poids de la composition finale, et de préférence à raison de 4 à 25 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium. Les pigments peuvent être enrobés ou non.

Les nacres peuvent être présentes dans la composition à raison de 0 à 20 % du poids total de la composition, de préférence à un taux de l'ordre de 2 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0 à 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple) et les fibres par exemple de polyamides.

Avantageusement, la composition est exempte de charges absorbantes comme le talc, la silice, le kaolin, les poudres de nylon. En revanche, elle peut contenir des charges non absorbantes à propriétés de diffusion optique de la lumière.

L'organopolysiloxane à groupement(s) oxyethyléné(s) présente l'avantage de bien disperser les pigments et de conférer ainsi, un maquillage homogène. Il présente en outre, l'avantage de stabiliser de façon remarquable les compositions en particulier lorsque celles-ci sont sous forme d'émulsion.

La composition selon l'invention peut être fabriquée à froid ou par chauffage d'un ou plusieurs organopolysiloxanes élastomériques sous forme de gel anhydre, ajout d'un ou plusieurs pigments et/ou d'un ou plusieurs autres additifs, ajout éventuel de corps gras fondus (notamment portée à la température de fusion des cires la plus élevée), ajout éventuel de la phase aqueuse, puis émulsification si nécessaire.

Elle peut aussi être obtenue par extrusion comme décrit dans la demande EP-A-667 146. Ce procédé consiste à malaxer la pâte (cires + huiles + eau + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention a encore pour objet l'utilisation de particules d'organopolysiloxane solide élastomérique réticulé comportant au moins un groupement d'oxyalkylène notamment d'oxyéthylène dans une composition cosmétique, telle que définie dans la revendication 22.

Avantageusement, la composition est exempte de résine de silicone en vue de conserver les propriétés de confort à l'application et au cours du temps.

L'invention a encore pour objet un procédé cosmétique pour augmenter l'apport de matité et/ou la tenue et/ou la résistance au sébum et/ou pour diminuer le transfert et/ou la migration d'une composition cosmétique, tel que défini dans la revendication 21. L'invention est illustrée plus en détail dans les exemples suivants. Les pourcentages sont donnés en poids.

### Exemple 1 : Réalisation d'un font de teint matifiant à texture « flan »

- Phase grasse
   ◆ Huile de silicone (PDMS, 6 cSt) 10%
   ◆ Pigment 10%
   ◆ Silicone modifiée (Exemple 3 du brevet US-A-5412004° 18% (5,7 % en matière active)
- Phase aqueuse
   ◆ Conservateur qs
   ◆ Sel divalent 0,4%
   ◆ Eau qsp 100%

### Résultat :

Obtention d'un fond de teint de texture gel type « flan » (dessert), très matifiant, d'une grande fraîcheur à l'application, de bonne tenue dans le temps et conservant un effet mat dans le temps, contrairement aux produits de l'art antérieur. Le fond de teint présente en outre des propriétés de non transfert et de non migration.

### Préparation :

On fait gonfler l'organopolysiloxane dans l'huile à température ambiante ensuite on ajoute les pigments, la phase aqueuse, puis on mélange le tout sous agitation, jusqu'à émulsification de la phase huileuse dans la phase aqueuse.

### Exemple 2 : Réalisation d'une crème de soin matifiante

- Phase grasse
   ◆ Silicone modifiée (Exemple du brevet US-A-5412004) 18%
   ◆ Huile de silicone (PDMS, 6cSt) 10%
- Phase aqueuse
   ◆ Conservateur qs
   ◆ Sel divalent 0,7%
   ◆ Eau qsp 100%

### Résultat :

Obtention d'une crème non grasse de consistance type « entremet » fraîche, très mate, de bonne tenue dans le temps. Cette crème contient plus de 70 % d'eau, sans ajout de tensioactif autre que l'élastomère, ni de co-émulsionnant.

### Préparation :

On prépare cette composition comme dans l'exemple 1.

Cette crème a été testée en comparaison avec une crème de l'art antérieur à base de KSG 6 et un autre à base de KSG 21. Elle a été jugée très mate alors que les autres crèmes de l'art antérieur ont été jugées brillantes. En outre, ses propriétés de non transfert et de non migration sont supérieures à celles du KSG 6.

### Test comparatif:

Un produit commercial Eclamat de Phas, sans organopolysiloxane conforme à l'invention et considéré par l'homme du métier, ici un cosméticien, comme un produit matifiant a été comparé à une composition de l'invention.

Ce test comparatif a été réalisé sur 16 femmes à peau grasse et brillante sur le front par demi-visage (une zone traitée du front contre une zone non traitée). La mesure de la brillance cutanée a été effectuée à l'aide d'un appareil du type Matidiag SEI-M-0029-MATI01 tel que décrit dans le document FR-A-2650890. Les mesures sont effectuées au temps T0, T15 minutes, T1 heure, T4 heures et T6 heures.

| Traitements | T15 mintutes - T0 | T1 heure - T0 | T4 heures - T0 | T6 heures -T0 |
|---|---|---|---|---|
| Invention | - 18% | - 16 % | -13 % | -10% |
| Eclamat | - 7% | - 5% | - 4% | -4% |

Il ressort clairement de ce tableau que l'organopolysiloxane à groupement oxyalkyléné confère un aspect matifiant significativement supérieur à celui du produit de l'art antérieur. Cette supériorité apparaît même de façon importante à l'oeil nu. En effet, plus la valeur mesurée est négative plus le produit est matifiant. En outre, cette matité tient bien dans le temps.

## Revendications

1. Utilisation cosmétique de particules d'organopolysiloxane solide élastomérique réticulé comportant au moins un groupement oxyalkylène et notamment oxyéthylèné dans une composition de maquillage contenant des pigments et une phase grasse liquide, comme agent pour matifier la peau ou les lèvres d'êtres humains et/ou camoufler les imperfections de la peau et/ou des lèvres, les dites particules servant comme agent tensioactif et épaississant de ladite phase grasse liquide.

2. Utilisation selon la revendication 1, **caractérisée en ce que** l'organopolysiloxane élastomérique comporte au moins un groupement d'oxyéthylène.

3. Utilisation selon l'une des revendications précédentes, **caractérisée en ce que** l'organopolysiloxane élastomérique ne comporte que des groupements oxyéthylénés comme groupements oxyalkylénés.

4. Utilisation selon l'une des revendications précédentes, **caractérisée en ce que** l'organopolysiloxane élastomérique est obtenu par réaction d'addition et de réticulation en milieu non aqueux, en présence notamment d'un catalyseur du type platine, d'au moins :
- un premier organopolysiloxane (i) ayant au moins deux groupements vinyliques en position α-ω de la chaîne siliconée par molécule ; et
- d'un second organopolysiloxane (ii) ayant au moins un atome d'hydrogène lié à un atome de silicium par molécule et au moins un groupement oxyalkyléné et notamment oxyéthylèné.

5. Utilisation selon la revendication 4, **caractérisée en ce que** le premier organopolysiloxane (i) est choisi parmi les polydiméthylsiloxanes.

6. Utilisation selon l'une des revendications 4 ou 5, **caractérisée en ce que** le premier organopolysiloxane (i) est un α-ω-diméthylvinyl polydiméthylsiloxane.

7. Utilisation selon l'une des revendications 4 à 6, **caractérisée en ce que** le second organopolysiloxane (ii) est choisi parmi les polydiméthylsiloxanes comportant un ou plusieurs atomes d'hydrogène et un ou plusieurs groupements oxyalkylénés notamment oxyéthylènés, liés à un atome de silicium via un radical alkylène ayant de 1 à 22 atomes de carbone.

8. Utilisation selon l'une quelconque des revendications 4 à 7, **caractérisée en ce que** les particules d'organopolysiloxane sont sous forme d'un gel anhydre obtenu selon les étapes suivantes :
- (a) mélange du premier et second organopolysiloxanes (i) et (ii) ;
- (b) ajout de la phase grasse liquide au mélange de l'étape (a) ;
- (c) polymérisation du premier et second organopolysiloxanes (i) et (ii) en phase grasse liquide en présence d'un catalyseur de platine.

9. Utilisation selon l'une des revendications précédentes, **caractérisée en ce que** les particules d'organopolysiloxanes sont sous forme d'un gel à comportement viscoélastique à 1 Hz de valeur de contrainte de cisaillement telle que 800 Pa < G * ₚₗₐₜₑₐᵤ < 2 500 Pa avec un δ ₚₗₐₜₑₐᵤ de 10°, G * ₚₗₐₜₑₐᵤ représentant la consistance et δ ₚₗₐₜₑₐᵤ représentant l'élasticité.

10. Utilisation selon l'une des revendications précédentes, **caractérisée en ce que** les particules d'organopolysiloxanes on une taille allant de 3 à 200 µm et mieux de 3 à 50 µm.

11. Utilisation selon l'une quelconque des revendications précédentes, **caractérisée en ce que** la phase grasse liquide contient une ou plusieurs huiles liquides hydrocarbonées et/ou siliconées.

12. Utilisation selon l'une des revendications précédentes, **caractérisée en ce que** la composition contient, entre autre, au moins un corps gras choisi parmi les huiles volatiles ou non, les cires, les gommes et les corps gras pâteux, d'origine animale, végétale, minérale ou synthétique, et leurs mélanges.

13. Utilisation selon l'une des revendications précédentes, **caractérisée en ce que** l'organopolysiloxane élastomérique représente de 0,1 à 33 % du poids total de la composition de préférence de 1 à 25 %.

14. Utilisation selon l'une des revendications précédentes, **caractérisée en ce qu'**elle comprend, en outre, une phase aqueuse représentant de 0 à 75 % et mieux de 5 à 60 % du poids total de la composition.

15. Utilisation selon l'une des revendications précédentes, **caractérisée en ce que** la phase grasse liquide représente de 1 à 80 % du poids total de la composition, de préférence de 1 à 50 %.

16. Utilisation selon l'une des revendications précédentes, **caractérisée en ce qu'**elle comprend, en outre, une phase particulaire présente à raison de 0 à 60 % du poids total de la composition, de préférence 5 à 35 %.

17. Utilisation selon l'une des revendications précédentes, **caractérisée en ce qu'**elle contient, en outre, au moins un actif cosmétique ou dermatologique.

18. Utilisation selon l'une des revendications précédentes, **caractérisée en ce qu'**elle est exempte de charge absorbantes et/ou de tensioactif autre que l'organopolysiloxane élastomérique.

19. Utilisation selon l'une des revendications précédentes, **caractérisée en ce qu'**elle se présente sous forme d'une composition de fond de teint, de fard à joues ou à paupières, d'un produit anti-cemes, d'un rouge à lèvres, d'un eye-liner, d'un mascara, d'un vernis à ongles, d'un produit de maquillage.

20. Utilisation selon l'une des revendications précédentes, **caractérisée en ce qu'**elle contient, en outre, au moins un ingrédient choisi parmi les conservateurs, les parfums, les gélifiants de phase aqueuse, les électrolytes.

21. Procédé cosmétique pour augmenter l'apport de matité et/ou la tenue et/ou la résistance au sébum et/ou pour diminuer le transfert et/ou la migration d'une composition cosmétique de maquillage contenant des pigments et une phase grasse liquide, consistant à introduire dans la composition des particules d'un organopolysiloxane solide élastomérique réticulé comportant au moins un groupement oxyéthyléné, les dites particules servant comme agent tensioactif et épaississant de ladite phase grasse liquide.

22. Utilisation cosmétique de particules d'organopolysiloxane solide élastomérique réticulé comportant au moins un groupement d'oxyalkylène et notamment d'oxyéthylène dans une composition cosmétique de maquillage contenant des pigments et une phase grasse liquide, comme agent d'augmentation de la matité et comme :
- agent de diminution du transfert
- et/ou agent d'augmentation de la tenue
- et/ou de la résistance au sébum de ladite composition,
les dites particules servant comme agent tensioactif et épaississant de ladite phase grasse liquide.

## Claims

1. Cosmetic use of particles of a crosslinked elastomeric solid polyorganosiloxane comprising at least one oxyalkylene group, and in particular an oxyethylenated group, in a make-up composition containing pigments and a liquid fatty phase as an agent giving a matt effect to human skin or lips and/or for camouflaging the imperfections of the skin and/or the lips, the said particles serving as surfactant and thickener for the said liquid fatty phase.

2. Use according to Claim 1, **characterized in that** the elastomeric polyorganosiloxane comprises at least one oxyethylene group.

3. Use according to either of the preceding claims, **characterized in that** the elastomeric polyorganosiloxane comprises only oxyethylenated groups as oxyalkylenated groups.

4. Use according to one of the preceding claims, **characterized in that** the elastomeric polyorganosiloxane is obtained by addition reaction and crosslinking reaction in non-aqueous medium, in particular in the presence of a catalyst of platinum type, of at least:
- a first polyorganosiloxane (i) containing at least two vinyl groups in the α-ω position on the silicone chain per molecule; and
- a second polyorganosiloxane (ii) containing at least one hydrogen atom linked to one silicon atom per molecule and at least one oxyalkylenated group, in particular an oxyethylenated group.

5. Use according to Claim 4, **characterized in that** the first polyorganosiloxane (i) is chosen from polydimethylsiloxanes.

6. Use according to either of Claims 4 and 5, **characterized in that** the first polyorganosiloxane (i) is an α-ω-dimethylvinyl polydimethylsiloxane.

7. Use according to one of Claims 4 to 6, **characterized in that** the second polyorganosiloxane (ii) is chosen from polydimethylsiloxanes comprising one or more hydrogen atoms and one or more oxyalkylenated groups, in particular oxyethylenated groups, linked to a silicon atom via an alkylene radical containing from 1 to 22 carbon atoms.

8. Use according to any one of Claims 4 to 7, **characterized in that** the polyorganosiloxane particles are in the form of an anhydrous gel obtained according to the following steps:
- (a) mixing the first and second polyorganosiloxanes (i) and (ii);
- (b) adding the liquid fatty phase to the mixture from step (a) ;
- (c) polymerizing the first and second polyorganosiloxanes (i) and (ii) in a liquid fatty phase in the presence of a platinum catalyst.

9. Use according to any one of the preceding claims, **characterized in that** the polyorganosiloxane particles are in the form of a gel having viscoelastic behaviour at 1 Hz with a shear stress value such that 800 Pa < G*ₚₗₐₜₑ < 2 500 Pa with a δₚₗₐₜₑ of 10°, G*ₚₗₐₜₑ representing the consistency and δₚₗₐₜₑ representing the elasticity.

10. Use according to one of the preceding claims, **characterized in that** the polyorganosiloxane particles have a size ranging from 3 to 200 µm and better still from 3 to 50 µm.

11. Use according to any one of the preceding claims, **characterized in that** the liquid fatty phase contains one or more hydrocarbon-based and/or silicone liquid oils.

12. Use according to one of the preceding claims, **characterized in that** the composition contains, inter alia, at least one fatty substance chosen from volatile or non-volatile oils, waxes, gums and pasty fatty substances, of animal, plant, mineral or synthetic origin, and mixtures thereof.

13. Use according to one of the preceding claims, **characterized in that** the elastomeric polyorganosiloxane represents from 0.1 to 33% relative to the total weight of the composition, preferably from 1 to 25%.

14. Use according to one of the preceding claims, **characterized in that** it also comprises an aqueous phase representing from 0 to 75% and better still from 5 to 60% relative to the total weight of the composition.

15. Use according to one of the preceding claims, **characterized in that** the liquid fatty phase represents from 1 to 80% relative to the total weight of the composition, preferably from 1 to 50%.

16. Use according to one of the preceding claims, **characterized in that** it also comprises a particulate phase present in a proportion of from 0 to 60% relative to the total weight of the composition, preferably 5 to 35%.

17. Use according to one of the preceding claims, **characterized in that** it also contains at least one cosmetic or dermatological active agent.

18. Use according to one of the preceding claims, **characterized in that** it is free of absorbent filler and/or of surfactant other than the elastomeric polyorganosiloxane.

19. Use according to one of the preceding claims, **characterized in that** it is in the form of a foundation, face powder or eyeshadow composition, a concealer product, a lipstick, an eyeliner, a mascara, a nail varnish, a make-up product.

20. Use according to one of the preceding claims, **characterized in that** it also contains at least one ingredient chosen from preserving agents, fragrances, aqueous-phase gelling agents and electrolytes.

21. Cosmetic process for increasing the provision of a matt effect and/or the staying power and/or the resistance to sebum and/or for reducing the transfer and/or the migration of a cosmetic make-up composition containing pigments and a liquid fatty phase, which consists in introducing into the composition particles of a crosslinked elastomeric solid polyorganosiloxane comprising at least one oxyethylenated group, the said particles serving as surfactant and thickener for the said liquid fatty phase.

22. Cosmetic use of particles of a crosslinked elastomeric solid polyorganosiloxane comprising at least one oxyalkylene group, and in particular an oxyethylene group, in a cosmetic make-up composition containing pigments and a liquid fatty phase as an agent for increasing the matt effect and as:
- an agent for reducing the transfer of the said composition
- and/or an agent for increasing its staying power
- and/or its resistance to sebum,
the said particles serving as surfactant and thickener for the said liquid fatty phase.

## Patentansprüche

1. Kosmetische Verwendung von Partikeln aus vernetztem elastomerem festem Polyorganosiloxan, das mindestens eine Oxyalkylen-Gruppe und insbesondere Oxyethylen-Gruppe enthält, in einer Schminkzusammensetzung, die Pigmente und eine flüssige Fettphase enthält, als Mittel zum Mattieren der Haut oder der Lippen des Menschen und/oder zu Verdecken der Unvollkommenheiten der Haut und/oder der Lippen, wobei die Partikel als grenzflächenaktiver Stoff und Verdickungsmittel der flüssigen Fettphase dienen.

2. Verwendung nach Anspruch 1, **dadurch gekennzeichnet, daß** das elastomere Polyorganosiloxan mindestens eine Oxyethylen-Gruppe enthält.

3. Verwendung nach einem der vorhergehenden Ansprüche, **dadurch gekennzeichnet, daß** das elastomere Polyorganosiloxan ausschließlich Oxyethylen-Gruppen als Oxyalkylen-Gruppen enthält.

4. Verwendung nach einem der vorhergehenden Ansprüche, **dadurch gekennzeichnet, daß** das elastomere Polyorganosiloxan durch Additionsreaktion und Vernetzung mindestens
- eines ersten Polyorganosiloxans (i), das pro Molekül mindestens zwei Vinylgruppen in α,ω-Position der Siliconkette aufweist, und
- eines zweiten Polyorganosiloxans (ii), das mindestens ein Wasserstoffatom, das mit einem Siliciumatom verbunden ist, pro Molekül und mindestens eine Oxyalkylen-Gruppe und insbesondere Oxyethylen-Gruppe aufweist,
in nicht-wäßrigem Medium in Gegenwart insbesondere eines Katalysators vom Platin-Typ erhalten wird.

5. Verwendung nach Anspruch 4, **dadurch gekennzeichnet, daß** das erste Polyorganosiloxan (i) unter den Polydimethylsiloxanen ausgewählt wird.

6. Verwendung nach Anspruch 4 oder 5, **dadurch gekennzeichnet, daß** das erste Polyorganosiloxan (i) ein α,ω-Dimethylvinylpolydimethylsiloxan ist.

7. Verwendung nach einem der Ansprüche 4 bis 6, **dadurch gekennzeichnet, daß** das zweite Polyorganosiloxan (ii) unter den Polydimethylsiloxanen, die ein oder mehrere Wasserstoffatome und eine oder mehrere Oxyalkylen-Gruppen und insbesondere Oxyethylen-Gruppen enthalten, die mit einem Siliciumatom über einen Alkylenrest mit 1 bis 22 Kohlenstoffatomen verbunden sind, ausgewählt wird.

8. Verwendung nach einem der Ansprüche 4 bis 7, **dadurch gekennzeichnet, daß** die Polyorganosiloxan-Partikel in Form eines wasserfreien Gels vorliegen, das durch die folgenden Verfahrensschritte erhalten wird:
(a) Vermischen des ersten Polyorganosiloxans (i) mit dem zweiten Polyorganosiloxan (ii);
(b) Zugeben der flüssigen Fettphase zu dem Gemisch aus Schritt (a);
(c) Polymerisieren des ersten Polyorganosiloxans (i) und des zweiten Polyorganosiloxans (ii) in flüssiger Fettphase in Gegenwart eines Platin-Katalysators.

9. Verwendung nach einem der vorhergehenden Ansprüche, **dadurch gekennzeichnet, daß** die Partikel aus Polyorganosiloxanen in Form eines Gels mit viskoelastischem Verhalten bei einer Scherbeanspruchung mit einem Wert von 1 Hz vorliegen, so daß 800 Pa < G*_{Plateau} < 2500 Pa mit einem δ_{Plateau} von 10°, wobei G*_{Plateau} die Konsistenz darstellt und δ_{Plateau} die Elastizität bedeutet.

10. Verwendung nach einem der vorhergehenden Ansprüche, **dadurch gekennzeichnet, daß** die Partikel aus Polyorganosiloxanen eine Größe im Bereich von 3 bis 200 µm und besser 3 bis 50 µm aufweisen.

11. Verwendung nach einem der vorhergehenden Ansprüche, **dadurch gekennzeichnet, daß** die flüssige Fettphase ein oder mehrere flüssige Kohlenwasserstofföle und/oder Siliconöle enthält.

12. Verwendung nach einem der vorhergehenden Ansprüche, **dadurch gekennzeichnet, daß** die Zusammensetzung außerdem mindestens eine Fettsubstanz enthält, die unter den gegebenenfalls flüchtigen Ölen, den Wachsen, den Gummen und den pastösen Fettsubstanzen tierischer, pflanzlicher, mineralischer oder synthetischer Herkunft und ihren Gemischen ausgewählt wird.

13. Verwendung nach einem der vorhergehenden Ansprüche, **dadurch gekennzeichnet, daß** das elastomere Polyorganosiloxan 0,1 bis 33 % und vorzugsweise 1 bis 25 % des Gesamtgewichts der Zusammensetzung ausmacht.

14. Verwendung nach einem der vorhergehenden Ansprüche, **dadurch gekennzeichnet, daß** sie außerdem eine wäßrige Phase enthält, die in einem Anteil von 0 bis 75 % und besser 5 bis 60 % des Gesamtgewichts der Zusammensetzung enthalten ist.

15. Verwendung nach einem der vorhergehenden Ansprüche, **dadurch gekennzeichnet, daß** der Anteil der flüssigen Fettphase 1 bis 80 % und vorzugsweise 1 bis 50 % des Gesamtgewichts der Zusammensetzung entspricht.

16. Verwendung nach einem der vorhergehenden Ansprüche, **dadurch gekennzeichnet, daß** sie außerdem eine partikelförmige Phase enthält, die in einem Anteil von 0 bis 60 % und vorzugsweise 5 bis 35 % des Gesamtgewichts der Zusammensetzung enthalten ist.

17. Verwendung nach einem der vorhergehenden Ansprüche, **dadurch gekennzeichnet, daß** sie außerdem mindestens einen kosmetischen oder dermatologischen Wirkstoff enthält.

18. Verwendung nach einem der vorhergehenden Ansprüche, **dadurch gekennzeichnet, daß** sie außer dem elastomeren Polyorganosiloxan keine absorbierenden Füllstoffe und/oder grenzflächenaktiven Stoffe enthält.

19. Verwendung nach einem der vorhergehenden Ansprüche, **dadurch gekennzeichnet, daß** sie in Form einer Make-up-Zusammensetzung, eines Rouge oder eines Lidschattens, eines Produkts gegen Augenringe, eines Lippenstifts, eines Eyeliners, eines Mascaras, eines Nagellacks, eines Schminkprodukts vorliegt.

20. Verwendung nach einem der vorhergehenden Ansprüche, **dadurch gekennzeichnet, daß** sie außerdem mindestens einen Bestandteil enthält, der unter den Konservierungsmitteln, den Parfüms, den Gelbildnern der wäßrigen Phase, den Elektrolyten ausgewählt wird.

21. Kosmetisches Verfahren zur Verbesserung der erzielbaren Mattigkeit und/oder der Beständigkeit und/oder der Widerstandsfähigkeit gegenüber Sebum und/oder zur Verringerung des Transfers und/oder der Migration einer kosmetischen Schminkzusammensetzung, die Pigmente und eine flüssige Fettphase enthält, das darin besteht, in die Zusammensetzung Partikel aus einem vernetzten elastomeren festen Polyorganosiloxan einzubringen, das mindestens eine Oxyethylen-Gruppe enthält, wobei die Partikel als grenzflächenaktiver Stoff und Verdickungsmittel der flüssigen Fettphase dienen.

22. Kosmetische Verwendung von Partikeln aus einem vernetzten elastomeren festen Polyorganosiloxan, das mindestens eine Oxyalkylengruppe und insbesondere Oxyethylengruppe enthält, in einer kosmetischen Schminkzusammensetzung, die Pigmente und eine flüssige Fettphase enthält, als Mittel zur Verbesserung der Mattigkeit und als
- Mittel zur Verringerung des Transfers,
- und/oder Mittel zur Verbesserung der Beständigkeit,
- und/oder Mittel zur Verbesserung der Widerstandsfähigkeit gegen Sebum
der Zusammensetzung, wobei die Partikel als grenzflächenaktiver Stoff und Verdickungsmittel der flüssigen Fettphase dienen.
